# FASCICULE DE BREVET EUROPEEN

(11) **EP 0 864 320 B1**
(45) Date de publication et mention de la délivrance du brevet: **27.10.1999**
(21) Numéro de dépôt: 98400425.9
(22) Date de dépôt: 20.02.1998
(51) Int. Cl.: A61K 7/48, A61K 7/00

(54) **Emulsion huile-dans-eau stable, son procédé de fabrication et son utilisation dans les domaines cosmétique et dermatologique**
Stabile Öl-in-Wasser Emulsion, Verfahren zu ihrer Herstellung und ihre Verwendung in der Kosmetik und der Dermatologie
Stable oil-in-water emulsion, its process of manufacturing and its use in the cosmetical and dermatological fields

(30) Priorité: 13.03.1997 FR 9703017
(43) Date de publication de la demande: 16.09.1998
(73) Titulaire: L'OREAL, 75008 Paris (FR)
(72) Inventeur: Simonnet, Jean-Thierry, 75011 Paris (FR); Le Verge, Danielle, 91270 Vigneux/S/Seine (FR); Legret, Sylvie, 92320 Chatillon (FR); Hansenne, Isabelle, 75017 Paris (FR)
(74) Mandataire: Tezier Herman, Béatrice

(56) Documents cités:
- EP-A- 0 681 830
- EP-A- 0 688 561
- EP-A- 0 692 237
- FR-A- 2 681 248

## Description

L'invention se rapporte à une émulsion huile-dans-eau (H/E) fine et stable, contenant des particules de polymère ionique, et à son procédé de préparation. Cette émulsion peut constituer une composition destinée notamment au traitement par voie topique de la peau (corps, visage) et/ou des cheveux, en particulier une composition cosmétique ou dermatologique, notamment destinée à la protection solaire. L'invention se rapporte aussi à l'utilisation de l'émulsion selon l'invention pour la stabilisation photochimique des filtres solaires chimiques ainsi qu'à l'utilisation des particules de polymère ionique pour la stabilisation d'émulsion huile-dans-eau, fluide.

Pour diverses raisons liées en particulier à leur grand confort d'utilisation et à leur fraîcheur, les compositions cosmétiques, et en particulier celles destinées à la photoprotection de la peau contre les rayons UV-A et UV-B, appelées compositions solaires, se présentent le plus souvent sous la forme d'une émulsion du type huile-dans-eau, comportant une phase huileuse dispersée de manière homogène dans une phase aqueuse. Dans ces émulsions classiques, qui contiennent des agents émulsionnants (ou tensioactifs) et d'éventuels additifs cosmétiques, la taille des globules constituant la phase grasse est généralement supérieure à plusieurs microns. De telles émulsions peuvent avoir des propriétés cosmétiques (toucher huileux) et physiques (stabilité) insuffisantes.

Par ailleurs, on observe que, malgré la présence des agents émulsionnants (ou tensioactifs), certaines de ces émulsions présentent un manque de stabilité dans le temps, manque de stabilité se traduisant par l'apparition d'un phénomène de séparation (déphasage) entre les phases aqueuse et huileuse de l'émulsion. Cette instabilité nuit à la conservation des émulsions.

Aussi, pour éviter ce phénomène indésirable, il est souvent nécessaire de faire appel à des agents dits épaississants, que l'on introduit alors dans l'émulsion et dont la fonction première est de créer, au sein de la phase aqueuse, une matrice gélifiée servant à figer dans son réseau tridimensionnel les globules de la phase grasse, assurant ainsi un maintien mécanique de l'ensemble de l'émulsion. Toutefois, cette addition d'épaississant limite les formes galéniques possibles, en excluant notamment les compositions très fluides.

Or, on cherche de plus en plus à préparer des compositions fluides, notamment solaires, plus spécialement dans le but de disposer de produits facilement vaporisables souvent considérés par l'utilisateur comme plus faciles à appliquer que les crèmes.

Par ailleurs, la présence de tensioactifs nécessite le plus souvent de fabriquer l'émulsion à chaud, ce qui limite notamment la nature des actifs à introduire dans l'émulsion. En particulier, ce procédé exclut l'emploi d'actifs thermosensibles. Aussi, cherche-t-on, en plus de la fluidité, à s'affranchir des tensioactifs.

Certes, il est connu du document EP-A-0 692 237 de stabiliser des émulsions H/E par des particules creuses de polymère non-ionique. Toutefois, ce type de particules creuses, de nature non-ionique et de taille de l'ordre du micron, ne permet pas d'obtenir des émulsions très fines, dont les gouttelettes d'huile ont une taille inférieure à 500 nm. En outre, les particules décrites dans ce document nécessitent la présence d'un agent gélifiant pour améliorer la stabilité de la dispersion et ne permettent pas d'obtenir des émulsions très fluides.

Le brevet EP-A-0 681 830 décrit certes également une émulsion d'une phase huileuse dans une phase aqueuse, comportant des globules d'huile, caractérisée en ce qu'elle contient des particules de polymère ionique de PMMA et en ce que le rapport en poids des particules de polymère à la phase huileuse va de 1/5 à 1/40. Par contre, aucune précision concernant la taille des globules d'huile n'est donnée. Ce document décrit d'ailleurs une émulsion normale, et non une microémulsion.

Par ailleurs, le document FR-A-2,681,248 décrit des compositions contenant des nanoparticules encapsulant une huile. De telles particules peuvent être incorporées dans une émulsion déjà préparée, mais ne peuvent participer à la préparation et à la stabilisation d'une émulsion car elles peuvent s'ouvrir lors d'une agitation trop vive. Elles ne sont, notamment, pas aptes à l'obtention d'émulsions très fines, c'est-à-dire comportant des gouttelettes d'huile d'une taille inférieure à 500 nm.

En outre, les nanoparticules décrites dans ce document forment des capsules renfermant une huile et ont pour fonction de protéger l'huile dans la composition afin de ne la libérer que lors de l'application sur la peau. L'huile y est complètement encapsulée dans la pellicule des nanoparticules. Le but de la présente invention est d'obtenir une émulsion où des gouttelettes d'huile très fines ne sont pas encapsulées, mais sont réparties dans la phase aqueuse.

Dans l'émulsion selon l'invention, les particules de polymère sont à l'interface de l'eau et des gouttelettes d'huile sous forme particulaire, sans former une capsule fermée continue entourant chaque gouttelette d'huile comme c'est le cas des nanoparticules. Le but est d'ailleurs tout à fait différent, puisque les nanoparticules d'huile ont pour fonction de protéger l'huile dans la composition pour la libérer lors de l'application sur la peau, tandis que les particules de polymère dans l'invention ont pour fonction de permettre la stabilisation de l'émulsion, même sans addition de tensioactif.

La demanderesse a découvert, de manière inattendue, que l'utilisation de particules de polymère ionique permettait d'obtenir des émulsions fluides stables, ne présentant pas les inconvénients des émulsions de l'art antérieur et comportant des globules d'huile ayant une taille moyenne inférieure à 500 nanomètres (nm).

La présente invention a donc pour objet une émulsion d'une phase huileuse dans une phase aqueuse comportant des globules d'huile ayant un diamètre inférieur à 500 nanomètres, caractérisée en ce qu'elle contient des particules de polymère ionique et en ce que le rapport entre la quantité de particules de polymère et la quantité de phase huileuse va de 1/5 à 1/40.

On entend par "polymère ionique" aussi bien un homopolymère qu'un copolymère. Les polymères ont notamment pour but de disperser la phase huileuse dans la phase aqueuse.

Les émulsions selon l'invention présentent notamment l'avantage de pouvoir être très fluides tout en présentant une très bonne stabilité, même en l'absence d'agent gélifiant.

En plus des avantages mentionnés ci-dessus (fluidité, stabilité), l'utilisation des particules de polymère comme dispersant permet d'effectuer à froid l'étape de dispersion de la phase huileuse dans la phase aqueuse, ce qui est plus simple et moins coûteux que les procédés classiques réalisés le plus souvent à chaud quand on utilise des tensioactifs. La fabrication à froid permet par exemple l'introduction d'actifs thermosensibles sans risque de dégradation de ces actifs.

Avantageusement, l'émulsion de l'invention est exempte de tensioactif. Ainsi, du fait de l'absence de tensioactif, cette émulsion présente l'avantage de ne pas être irritante pour les peaux particulièrement sensibles.

Par ailleurs, l'émulsion ainsi obtenue est très fine et présente des qualités sensorielles particulièrement satisfaisantes. La taille moyenne des globules constituant la phase huileuse est inférieure à 500 nm, et elle va de préférence de 150 nm à 300 nm.

L'émulsion selon l'invention peut être très fluide, ce qui signifie qu'elle peut présenter une viscosité inférieure 15 000 cPs (soit 15 Pa.s), encore plus préférentiellement inférieure à 5 000 cPs (soit 5 Pa.s) (mesurée sur viscosimètre Brookfield RVT modèle DV2, à 0,5 tour/mn et avec disque n°5).

De façon générale, les particules utilisables dans l'invention peuvent être réalisées à partir d'un polymère ionique, d'un mélange de polymères ioniques ou d'un mélange d'au moins un polymère ionique et d'au moins un polymère non-ionique. Ces polymères doivent être non toxiques et non irritants pour la peau. En outre, ils doivent pouvoir se disperser dans l'eau sous forme particulaire.

Le polymère ionique peut être cationique ou anionique. Il s'agit de préférence d'un polymère anionique. Les polymères anioniques utilisables dans l'invention sont par exemple les polymères d'acide isophtalique ou d'acide sulfoisophtalique, et en particulier les copolymères de phtalate / sulfoisophtalate / glycol (par exemple diéthylèneglycol / Phtalate / isophtalate / 1,4-cyclohexane-diméthanol) vendus sous les dénominations "Eastman AQ polymer" (AQ35S, AQ38S, AQ55S, AQ48 Ultra) par la société Eastman Chemical.

De façon avantageuse, les particules de polymère ionique utilisées selon l'invention ont une granulométrie allant de 10 à 400 nm et encore mieux allant de 20 à 200 nm selon la nature du polymère ionique.

Les particules de ces polymères peuvent être utilisées telles quelles ou en dispersion dans l'eau.

Dans les émulsions de l'invention, on peut utiliser une quantité de particules de polymère allant de 0,1 à 10 %, de préférence de 0,5 à 5 % et mieux de 1 à 2 % du poids total de la composition.

Le rapport en poids des particules de polymère à la phase huileuse va avantageusement de 1/5 à 1/40, et de préférence de 1/10 à 1/20. Un tel rapport permet d'obtenir une dispersion d'huile fluide, fine, c'est-à-dire ayant des globules d'une granulométrie inférieure à 500 nm, et parfaitement stable.

L'invention a aussi pour objet l'utilisation de particules de polymère ionique pour la stabilisation d'une émulsion de type huile-dans-eau, fluide, comportant des globules d'huile ayant une taille moyenne d'au plus 500 nm.

L'émulsion selon l'invention peut être utilisée dans tous les domaines utilisant ce type de forme galénique et notamment dans les domaines cosmétique et dermatologique. Quand elle constitue une composition cosmétique et/ou dermatologique, elle contient, en outre, avantageusement un milieu physiologiquement acceptable. On entend par physiologiquement acceptable un milieu compatible avec la peau, les muqueuses, les ongles et les cheveux.

Les émulsions, objet de l'invention, trouvent leur application dans un grand nombre de traitements cosmétiques et/ou dermatologiques de la peau, des muqueuses et des cheveux, y compris le cuir chevelu, notamment pour la protection, le soin, le nettoyage et le maquillage de la peau et des muqueuses, pour la protection et le soin des cheveux et pour le traitement thérapeutique de la peau, des cheveux et des muqueuses et plus spécialement des lèvres.

Les émulsions selon l'invention peuvent par exemple être utilisées dans des produits de soin ou de nettoyage pour le visage sous forme de crèmes ou de laits ou comme produits de maquillage (peau et lèvres) par incorporation de charges, de pigments ou de colorants. Selon un mode particulier de réalisation de l'invention, l'émulsion contient un filtre solaire et constitue plus particulièrement une composition solaire destinée à la protection de la peau, des muqueuses et/ou des cheveux contre le rayonnement ultraviolet, en particulier contre le rayonnement solaire.

Aussi, l'invention a encore pour objet l'utilisation cosmétique de l'émulsion telle que définie ci-dessus pour le traitement de la peau, des muqueuses et/ou les cheveux et/ou pour la protection de la peau, des muqueuses et/ou des cheveux contre le rayonnement ultraviolet, en particulier le rayonnement solaire.

L'invention a aussi pour objet l'utilisation de l'émulsion telle que définie ci-dessus pour la fabrication d'une composition dermatologique destinée au traitement de la peau, des muqueuses et/ou des cheveux et/ou à la protection de la peau, des muqueuses et/ou des cheveux contre le rayonnement ultraviolet, en particulier le rayonnement solaire.

L'invention a encore pour objet un procédé de traitement cosmétique pour protéger la peau, des muqueuses et/ou les cheveux contre le rayonnement ultraviolet, en particulier le rayonnement solaire, caractérisé en ce qu'il consiste à appliquer sur la peau, les muqueuses et/ou les cheveux une quantité efficace de la composition solaire telle que définie ci-dessus.

Les compositions solaires contiennent un ou plusieurs filtres solaires actifs dans l'UV-A et/ou l'UV-B. Ces filtres peuvent être des filtres chimiques hydrophiles ou lipophiles ou des pigments. A titre d'exemples, les filtres chimiques peuvent être choisis parmi l'acide 2-phényl benzimidazole 5-sulfonique et ses sels, les dérivés cinnamiques tels que par exemple le p-méthoxycinnamate de 2-éthylhexyle, les dérivés salicyliques comme par exemple le salicylate de 2-éthylhexyle et le salicylate d'homomenthyle, les dérivés du camphre comme par exemple le 3-(4-méthylbenzylidène)-camphre ou l'acide camphosulfonique-(1,4-divinylbenzène), les dérivés de triazine tels que la 2,4,6-tris-[p-(2'-éthylhexyl-1'-oxycarbonyl)anilino]-1,3,5-triazine, les dérivés de la benzophénone tels que la 2-hydroxy-4-méthoxybenzophénone, les dérivés du dibenzoylméthane tels que le 4-tert-butyl 4'-méthoxydibenzoylméthane, les dérivés de β,β-diphénylacrylate tels que l'α-cyano-β,β-diphénylacrylate de 2-éthylhexyle ou octocrylène, les dérivés de l'acide p-aminobenzoïque comme par exemple le paradiméthylaminobenzoate d'octyle, l'anthranilate de menthyle. On peut aussi citer comme filtres les polymères filtres et silicones filtres décrits dans la demande WO-A-93/04665, et notamment les silicones benzotriazoles.

D'autres exemples de filtres organiques sont donnés dans la demande de brevet EP-A-0 487 404.

Lorsque les compositions solaires de l'invention contiennent des pigments, ces derniers peuvent être des pigments ou des nanopigments (taille moyenne des particules primaires : généralement entre 5 nm et 100 nm, de préférence entre 10 et 50 nm) d'oxydes métalliques, enrobés ou non, comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflexion et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A- 0 518 772 et EP-A- 0 518 773.

Ces filtres chimiques et/ou pigments peuvent être éventuellement présents dans des compositions autres que les compositions solaires quand une certaine protection solaire est souhaitée.

Par ailleurs, la demanderesse a trouvé aussi de manière inattendue que les émulsions selon l'invention permettaient une stabilisation photochimique des filtres solaires chimiques. Aussi, l'invention a encore pour objet l'utilisation de l'émulsion telle que définie ci-dessus pour la stabilisation photochimique de filtre solaires chimiques.

La nature de la phase huileuse rentrant dans la composition des émulsions selon l'invention n'est pas critique et elle peut ainsi être constituée par tous les corps gras et notamment les huiles, classiquement utilisées dans les domaines cosmétique et dermatologique.

Parmi les huiles utilisables dans l'émulsion de l'invention, on peut notamment citer par exemple les huiles végétales (jojoba, avocat), les huiles minérales (vaseline), les huiles de synthèse (palmitate d'éthylhexyle, myristate d'isopropyle), les huiles de silicone (cyclométhicone) et les huiles fluorées. Les autres corps gras susceptibles d'être présents dans la phase huileuse peuvent être par exemple les acides gras, les alcools gras et les cires (cire liquide de jojoba).

La phase huileuse de l'émulsion peut représenter de 0,1 à 45 % et mieux de 5 à 30 % du poids total de l'émulsion.

En outre, de façon connue, les émulsions de l'invention peuvent contenir, outre les filtres solaires et les pigments mentionnés ci-dessus, des adjuvants habituels dans le domaine cosmétique ou dermatologique, tels que des actifs hydrophiles ou lipophiles, des conservateurs, des antioxydants, des parfums, des charges, des matières colorantes et encore des vésicules lipidiques. Ces adjuvants sont utilisés dans les proportions habituelles dans le domaine cosmétique ou dermatologique, et par exemple de 0,01 à 20 % du poids total de l'émulsion, et ils sont, selon leur nature, introduits dans la phase aqueuse ou dans la phase huileuse de l'émulsion, ou encore dans des vésicules. Ces adjuvants ainsi que leurs concentrations doivent être tels qu'ils ne modifient pas la propriété recherchée pour l'émulsion.

Si l'on souhaite obtenir une émulsion moins fluide, on peut y ajouter un ou plusieurs gélifiants comme les argiles, les gommes polysaccharides (gomme de xanthane), les polymères carboxyvinyliques ou carbomers. Ces gélifiants sont utilisés à des concentrations allant de 0,1 à 10 %, de préférence 0,1 à 5 % et mieux de 0,1 à 3 % du poids total de la composition.

Les émulsions de l'invention peuvent éventuellement être exemptes de solvant. Ceci va aussi dans le sens d'une émulsion peu agressive et non irritante susceptible d'être utilisée par des personnes à peau sensible. Toutefois, si nécessaire, elles peuvent contenir un solvant, notamment un alcool inférieur comportant de un à six atomes de carbone, plus particulièrement l'éthanol. La quantité de solvant peut aller jusqu'à 30 % du poids total de la composition.

Les émulsions selon l'invention peuvent être préparées par tout moyen approprié comportant une étape d'homogénéisation sous pression. Selon un mode préféré de réalisation, la préparation est faite en deux étapes.

Aussi, l'invention a encore pour objet un procédé de fabrication de l'émulsion, consistant dans une première étape à mélanger sous agitation la phase aqueuse, la phase huileuse et les particules de polymère et, dans une seconde étape à soumettre le mélange obtenu à une homogénéisation basée sur le principe de la cavitation.

Dans la première étape, le mélange est soumis à agitation classique par exemple dans un homogénéisateur tournant à une vitesse comprise entre 500 et 5000 tours/mn pendant un temps compris entre 10 et 60 mn environ à une température comprise entre 20 et 95°C environ.

L'homogénéisation basée sur le principe de la cavitation de la deuxième étape est une étape clé du procédé selon l'invention. Cette homogénéisation résulte du phénomène de cavitation créé et entretenu au sein du mélange, alors sous forme liquide, en mouvement à une vitesse linéaire d'au moins 100m/s.

Cette homogénéisation peut être opérée par utilisation d'un homogénéisateur haute pression fonctionnant sous une pression allant de 100 à 1000 bars environ, et de préférence de 400 à 700 bars. Le principe d'utilisation de ce type d'homogénéisateur est bien connu de l'homme de l'art. On opère à la température ambiante par passages successifs, généralement de 2 à 10 passages, sous la pression retenue, le mélange étant ramené à la température ambiante entre chaque passage.

L'homogénéisation peut également être obtenue sous l'action d'ultrasons ou encore par utilisation d'homogénéisateurs équipés d'une tête de type rotor-stator.

Les exemples suivants illustrent l'invention. Dans ces exemples, les pourcentages sont donnés en poids.

### Exemple 1 : Composition fluide solaire

| *Phase A* | |
|---|---|
| AQ38S (Eastman Chemical) | 2 % |
| Glycérine | 5 % |
| Conservateurs | 1,2 % |
| Séquestrant | 0,1 % |
| Eau déminéralisée | qsp 100 % |

| *Phase B* | |
|---|---|
| Octocrylène | 10 % |
| 4 tertiobutyl 4' méthoxy dibenzoylméthane (Parsol 1789 vendu par la société Givaudan) | 2 % |
| Cyclométhicone | 4 % |
| Cire liquide de jojoba (Flora Tech) | 4 % |

Mode opératoire : On mélange les constituants de la phase A et on chauffe le mélange à 70°C sous agitation magnétique jusqu'à dispersion complète du polymère, puis on refroidit la solution jusqu'à température ambiante. On prépare par ailleurs la phase B.

On introduit la phase A dans la phase B sous vive agitation. On homogénéise l'émulsion sous une pression de 600 bars (2 à 4 passages) en ramenant l'émulsion à la température ambiante entre chaque passage.

On obtient une émulsion fluide, apte à protéger la peau contre les rayons du soleil.

### Exemple 2 : Composition solaire

| *Phase A* | |
|---|---|
| AQ38S (Eastman Chemical) | 2 % |
| Glycérine | 5 % |
| Conservateurs | 1,2 % |
| Séquestrant | 0,1 % |
| Eau déminéralisée | 34,7 % |

| *Phase B* | |
|---|---|
| Octocrylène | 10 % |
| 4 tertiobutyl 4' méthoxy dibenzoylméthane (Parsol 1789 vendu par la société Givaudan) | 2 % |
| Cyclométhicone | 4 % |
| Cire liquide de jojoba (Flora Tech) | 4 % |

| *Phase C* | |
|---|---|
| Gomme de xanthane | 0,5 % |
| Eau déminéralisée | qsp 100 % |

Le mode opératoire est identique à celui de l'exemple 1 en ajoutant la phase C après le mélange des phases A et B et avant passage à l'homogénéisateur.

La photostabilité de l'octocrylène dans les compositions de ces exemples a été étudiée comparativement à une composition classique (filtre dans huile de colza) contenant la même quantité de filtre, dans le test suivant :

Pour chacune de ces compositions, on a déterminé le pourcentage d'octocrylène résiduel après irradiation par des UV selon le protocole suivant : pour chaque formule, on a préparé quatre échantillons témoins et quatre échantillons tests. On a déposé sur des plaques de PMMA (polyméthylméthacrylate) dépolies, préalablement rincées à l'eau puis séchées, 16 mg de formule qu'on a étalée sur une surface de 2 x 4 cm². On a ensuite laissé reposer toutes les plaques une demi-heure à l'obscurité. Puis on a irradié les plaques (SUNTEST CPS Heraeus) pendant 4 heures et 5 minutes en conservant les plaques témoins à l'obscurité pendant le temps d'irradiation des autres plaques.

On a ensuite dosé les échantillons de la manière suivante : on a procédé à l'extraction du filtre en immergeant chaque plaque dans 50 g d'isopropanol afin de solubiliser le filtre. Les plaques et le solvant contenant le filtre ont ensuite été traités aux ultrasons pendant 5 minutes pour assurer une agitation efficace. La concentration totale en filtre résiduel a été dosée au spectrophotomètre.

Les résultats en pourcentage de filtre résiduel sont consignés dans le tableau I suivant :

**Tableau I**

| Composition | % d'octocrylène résiduel |
|---|---|
| Composition classique | 92 % |
| Exemple 1 | 99,6 % |
| Exemple 2 | 99,7 % |

Ces résultats montrent clairement que la photostabilité de l'octocrylène est augmentée dans les émulsions de l'invention.

### Exemple 3 : Produit de soin hydratant

| *Phase A* | |
|---|---|
| AQ38S (Eastman Chemical) | 2 % |
| Glycérine | 5 % |
| Eau déminéralisée | qsp 100 % |

| *Phase B* | |
|---|---|
| Huile d'avocat | 7 % |
| Huile de jojoba | 7 % |
| Conservateur | 0,1 % |
| Cyclométhicone | 6 % |

Le mode opératoire est identique à celui de l'exemple 1.

On obtient une émulsion très fluide, blanche où la taille moyenne de globules d'huile est inférieure à 350 nm.

### Exemple 4 : Produit de soin hydratant

| *Phase A* | |
|---|---|
| AQ55S (Eastman Chemical) | 2 % |
| Glycérine | 5 % |
| Conservateurs | 1 % |
| Eau déminéralisée | 35 % |

| *Phase B* | |
|---|---|
| Huile d'avocat | 7 % |
| Huile de jojoba | 7 % |
| Cyclométhicone | 6 % |

| *Phase C* | |
|---|---|
| Gomme de xanthane | 0,5 % |
| Eau déminéralisée | qsp 100 % |

Le mode opératoire est identique à celui de l'exemple 2.

On obtient une émulsion très fine où la taille moyenne de globules d'huile est de l'ordre de 230 nm.

### Exemple 5 : Fluide après-soleil

| *Phase A* | |
|---|---|
| AQ48 Ultra (Eastman Chemical) | 2 % |
| Glycérine | 5 % |
| Conservateurs | 1 % |
| Eau déminéralisée | qsp 100 % |

| *Phase B* | |
|---|---|
| Huile de jojoba | 10 % |
| Cyclométhicone | 10 % |
| Alpha-bisabolol (agent apaisant) | 0,25 % |

Le mode opératoire est le même que celui de l'exemple 1. On obtient une émulsion très fluide où la taille moyenne de globules d'huile est de l'ordre de 225 nm.

### Exemple 6 : Lait démaquillant

| *Phase A* | |
|---|---|
| AQ38S (Eastman Chemical) | 2 % |
| Glycérine | 5 % |
| Conservateurs | 1 % |
| Eau déminéralisée | 35 % |

| *Phase B* | |
|---|---|
| Palmitate d'éthylhexyle | 14 % |
| Myristate d'isopropyle | 10 % |
| Cyclométhicone | 6 % |

| *Phase C* | |
|---|---|
| Gomme de xanthane | 0,3 % |
| Eau déminéralisée | qsp 100 % |

Le mode opératoire est le même que celui de l'exemple 2. On obtient une émulsion très fluide où la taille moyenne de globules d'huile est de l'ordre de 250 nm. Cette émulsion a de très bonnes propriétés démaquillantes.

## Revendications

1. Emulsion d'une phase huileuse dans une phase aqueuse, comportant des globules d'huile ayant un diamètre inférieur à 500 nanomètres, caractérisée en ce qu'elle contient des particules de polymère ionique et en ce que le rapport en poids des particules de polymère à la phase huileuse va de 1/5 à 1/40.

2. Emulsion selon la revendication 1, caractérisée en ce qu'elle est exempte de tensioactif.

3. Emulsion selon la revendication 1 ou 2, caractérisée en ce que les particules de polymère ionique sont réalisées à partir d'un polymère ionique, d'un mélange de polymères ioniques ou d'un mélange d'au moins un polymère ionique et d'au moins un polymère non-ionique.

4. Emulsion selon l'une quelconque des revendications précédentes, caractérisée en ce que le polymère ionique est un polymère anionique.

5. Emulsion selon la revendication 4, caractérisée en ce que le polymère ionique est un copolymère d'acide isophtalique et/ou d'acide sulfoisophtalique.

6. Emulsion selon l'une quelconque des revendications précédentes, caractérisée en ce que les particules ont une granulométrie allant de 10 à 400 nanomètres.

7. Emulsion selon l'une quelconque des revendications précédentes, caractérisée en ce que les particules représentent de 0,1 à 10 % du poids total de l'émulsion.

8. Emulsion selon l'une quelconque des revendications précédentes, caractérisée en ce que le rapport en poids des particules de polymère à la phase huileuse va de 1/10 à 1/20.

9. Emulsion selon l'une quelconque des revendications précédentes, caractérisée en ce que la phase huileuse représente de 0,1 à 45 % du poids total de l'émulsion.

10. Emulsion selon l'une quelconque des revendications précédentes, caractérisée en ce qu'elle est une composition cosmétique et/ou dermatologique.

11. Emulsion selon l'une quelconque des revendications précédentes, caractérisée en ce quelle contient au moins un additif choisi parmi les actifs hydrophiles, les actifs lipophiles, les conservateurs, les antioxydants, les parfums, les charges, les filtres solaires, les pigments, les matières colorantes et les vésicules lipidiques.

12. Composition solaire, caractérisée en ce qu'elle comporte l'émulsion selon l'une quelconque des revendications précédentes et au moins un filtre solaire.

13. Composition solaire selon la revendication 12, caractérisée en ce que le filtre solaire est un filtre chimique.

14. Composition solaire selon la revendication 13, caractérisée en ce que le filtre solaire est choisi parmi l'acide 2-phényl benzimidazole 5-sulfonique et ses sels, les dérivés cinnamiques , les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, l'anthranilate de menthyle, les polymères filtres et les silicones filtres.

15. Composition solaire selon l'une quelconque des revendications 12 à 14, caractérisée en ce que le filtre solaire est un pigment ou un nanopigment.

16. Utilisation cosmétique de l'émulsion selon l'une quelconque des revendications 1 à 15 pour le traitement de la peau, des muqueuses et/ou les cheveux et/ou pour la protection de la peau, des muqueuses et/ou des cheveux contre le rayonnement ultraviolet, en particulier le rayonnement solaire.

17. Utilisation de l'émulsion selon l'une quelconque des revendications 1 à 15 pour la fabrication d'une composition dermatologique destinée au traitement de la peau, des muqueuses et/ou les cheveux et/ou à la protection de la peau, des muqueuses et/ou des cheveux contre le rayonnement ultraviolet, en particulier le rayonnement solaire.

18. Procédé de traitement cosmétique pour protéger la peau, les muqueuses et/ou les cheveux contre le rayonnement ultraviolet, en particulier le rayonnement solaire, caractérisé en ce qu'il consiste à appliquer sur ceux-ci une quantité efficace de la composition selon l'une quelconque des revendications 12 à 15.

19. Procédé de fabrication d'une émulsion telle que définie dans les revendications 1 à 11, consistant dans une première étape à mélanger sous agitation la phase aqueuse, la phase huileuse et les particules de polymère et, dans une seconde étape à soumettre le mélange obtenu à une homogénéisation basée sur le principe de la cavitation.

20. Procédé de fabrication selon la revendication 19, caractérisé en ce que dans la seconde étape, l'homogénéisation est réalisée sous une pression allant de 400 à 700 bars.

21. Utilisation de particules de polymère ionique pour la stabilisation d'une émulsion de type huile-dans-eau, fluide comportant des globules d'huile ayant une taille moyenne d'au plus 500 nanomètres.

22. Utilisation de l'émulsion selon l'une quelconque des revendications 1 à 11, pour la stabilisation photochimique de filtre solaire chimique.

23. Utilisation selon la revendication 22, caractérisée en ce que le filtre est l'α-cyano-β,β-diphénylacrylate de 2-éthylhexyle.

## Claims

1. Emulsion of an oily phase in an aqueous phase containing oil globules having a diameter of less than 500 nanometres, characterized in that it contains ionic polymer particles and in that the ratio by weight of the polymer particles to the oily phase ranges from 1/5 to 1/40.

2. Emulsion according to Claim 1, characterized in that it is devoid of surfactant.

3. Emulsion according to Claim 1 or 2, characterized in that the ionic polymer particles are produced from an ionic polymer, from a mixture of ionic polymers or from a mixture of at least one ionic polymer and of at least one non-ionic polymer.

4. Emulsion according to any one of the preceding claims, characterized in that the ionic polymer is an anionic polymer.

5. Emulsion according to Claim 4, characterized in that the ionic polymer is a copolymer of isophthalic acid and/or of sulphoisophthalic acid.

6. Emulsion according to any one of the preceding claims, characterized in that the particles have a particle size ranging from 10 to 400 nanometres.

7. Emulsion according to any one of the preceding claims, characterized in that the particles represent from 0.1 to 10% of the total weight of the emulsion.

8. Emulsion according to any one of the preceding claims, characterized in that the ratio by weight of the polymer particles to the oily phase ranges from 1/10 to 1/20.

9. Emulsion according to any one of the preceding claims, characterized in that the oily phase represents from 0.1 to 45% of the total weight of the emulsion.

10. Emulsion according to any one of the preceding claims, characterized in that it is a cosmetic and/or dermatological composition.

11. Emulsion according to any one of the preceding claims, characterized in that it contains at least one additive chosen from hydrophilic active principles, lipophilic active principles, preservatives, antioxidants, fragrances, fillers, sunscreens, pigments, colouring materials and lipid vesicles.

12. Anti-sun composition, characterized in that it contains the emulsion according to any one of the preceding claims and at least one sunscreen.

13. Anti-sun composition according to Claim 12, characterized in that the sunscreen is a chemical screening agent.

14. Anti-sun composition according to Claim 13, characterized in that the sunscreen is chosen from 2-phenylbenzimidazole-5-sulphonic acid and its salts, cinnamic derivatives, salicylic derivatives, camphor derivatives, triazine derivatives, benzophenone derivatives, dibenzoylmethane derivatives, β,β-diphenylacrylate derivatives, p-aminobenzoic acid derivatives, menthyl anthranilate, screening polymers and screening silicones.

15. Anti-sun composition according to any one of Claims 12 to 14, characterized in that the sunscreen is a pigment or a nanopigment.

16. Cosmetic use of the emulsion according to any one of Claims 1 to 15, for the treatment of the skin, mucous membranes and/or hair and/or for the protection of the skin, mucous membranes and/or hair against ultraviolet radiation, in particular solar radiation.

17. Use of the emulsion according to any one of Claims 1 to 15 for the manufacture of a dermatological composition intended for the treatment of the skin, mucous membranes and/or hair and/or for the protection of the skin, mucous membranes and/or hair against ultraviolet radiation, in particular solar radiation.

18. Cosmetic treatment process for protecting the skin, mucous membranes and/or hair against ultraviolet radiation, in particular solar radiation, characterized in that it consists in applying an effective amount of the composition according to any one of Claims 12 to 15 to the latter.

19. Process for the manufacture of an emulsion as defined in Claims 1 to 11 which consists, in a first stage, in mixing the aqueous phase, the oily phase and the polymer particles with stirring and, in a second stage, in subjecting the mixture obtained to a homogenization based on the cavitation principle.

20. Manufacturing process according to Claim 19, characterized in that, in the second stage, the homogenization is carried out under a pressure ranging from 400 to 700 bar.

21. Use of ionic polymer particles for the stabilization of a fluid emulsion of oil-in-water type containing oil globules having a mean size of at most 500 nanometres.

22. Use of the emulsion according to any one of Claims 1 to 11 for the photochemical stabilization of a chemical sunscreen.

23. Use according to Claim 22, characterized in that the screening agent is 2-ethylhexyl α-cyano-β,β-diphenylacrylate.

## Patentansprüche

1. Emulsion einer Ölphase in einer wässerigen Phase, die Ölkügelchen mit einem Durchmesser unter 500 Nanometer enthält, dadurch gekennzeichnet, daß sie Partikel eines ionischen Polymers enthält, und dadurch, daß das Gewichtsverhältnis der Polymerpartikel zu der Ölphase im Bereich von 1/5 bis 1/40 liegt.

2. Emulsion nach Anspruch 1, dadurch gekennzeichnet, daß sie keinen grenzflächenaktiven Stoff enthält.

3. Emulsion nach Anspruch 1 oder 2, dadurch gekennzeichnet, daß die Partikel des ionischen Polymers aus einem ionischen Polymer, einem Gemisch von ionischen Polymeren oder aus einem Gemisch von mindestens einem ionischen Polymer und mindestens einem nichtionischen Polymer hergestellt sind.

4. Emulsion nach einem der vorhergehenden Ansprüche, dadurch gekennzeichnet, daß das ionische Polymer ein anionisches Polymer ist.

5. Emulsion nach Anspruch 4, dadurch gekennzeichnet, daß das ionische Polymer ein Copolymer von Isophthalsäure und/oder Sulfoisophthalsäure ist.

6. Emulsion nach einem der vorhergehenden Ansprüche, dadurch gekennzeichnet, daß die Partikel eine Größe von 10 bis 400 Nanometer aufweisen.

7. Emulsion nach einem der vorhergehenden Ansprüche, dadurch gekennzeichnet, daß die Partikel 0,1 bis 10 % des Gesamtgewichts der Emulsion ausmachen.

8. Emulsion nach einem der vorhergehenden Ansprüche, dadurch gekennzeichnet, daß das Gewichtsverhältnis der Polymerpartikel zu der Ölphase im Bereich von 1/10 bis 1/20 liegt.

9. Emulsion nach einem der vorhergehenden Ansprüche, dadurch gekennzeichnet, daß die Ölphase 0,1 bis 45 % des Gesamtgewichts der Emulsion ausmacht.

10. Emulsion nach einem der vorhergehenden Ansprüche, dadurch gekennzeichnet, daß sie eine kosmetische und/oder dermatologische Zusammensetzung ist.

11. Emulsion nach einem der vorhergehenden Ansprüche, dadurch gekennzeichnet, daß sie mindestens einen Zusatzstoff enthält, der unter hydrophilen Wirkstoffen, lipophilen Wirkstoffen, Konservierungsmitteln, Antioxidantien, Parfums, Füllstoffen, Sonnenschutzfiltern, Pigmenten, Färbemitteln und Lipidvesikeln ausgewählt ist.

12. Sonnenschutzzusammensetzung, dadurch gekennzeichnet, daß sie eine Emulsion nach einem der vorhergehenden Ansprüche und mindestens ein Sonnenschutzfilter enthält.

13. Zusammensetzung nach Anspruch 12, dadurch gekennzeichnet, daß das Sonnenschutzfilter ein chemisches Filter ist.

14. Sonnenschutzzusammensetzung nach Anspruch 13, dadurch gekennzeichnet, daß das Sonnenschutzfilter unter 2-Phenylbenzimidazol-5-sulfonsäure und ihren Salzen, Zimtsäurederivaten, Salicylsäurederivaten, Campherderivaten, Triazinderivaten, Benzophenonderivaten, Dibenzoylmethanderivaten, β,β-Diphenylacrylatderivaten, p-Aminobenzoesäurederivaten, Menthylanthranilat, Polymerfiltern und Siliconfiltern ausgewählt ist.

15. Sonnenschutzzusammensetzung nach einem der Ansprüche 12 bis 14, dadurch gekennzeichnet, daß das Sonnenschutzfilter ein Pigment oder ein Nanopigment ist.

16. Kosmetische Verwendung der Emulsion nach einem der Ansprüche 1 bis 15 zur Behandlung der Haut, der Schleimhäute und/oder der Haare und/oder zum Schutz der Haut, der Schleimhäute und/oder der Haare gegenüber ultravioletter Strahlung und insbesondere gegenüber Sonnenlicht.

17. Verwendung der Emulsion nach einem der Ansprüche 1 bis 15 zur Herstellung einer dermatologischen Zusammensetzung, die zur Behandlung der Haut, der Schleimhäute und/oder der Haare und/oder zum Schutz der Haut, der Schleimhäute und/oder der Haare gegenüber ultravioletter Strahlung und insbesondere gegenüber Sonnenlicht bestimmt ist.

18. Kosmetisches Behandlungsverfahren zum Schutz der Haut, der Schleimhäute und/oder der Haare gegenüber ultravioletter Strahlung und insbesondere gegenüber Sonnenlicht, das darin besteht, eine wirksame Menge der Zusammensetzung nach einem der Ansprüche 12 bis 15 auf die Haut, die Schleimhäute und/oder die Haare aufzutragen.

19. Verfahren zur Herstellung einer in einem der Ansprüche 1 bis 11 definierten Emulsion, das darin besteht, in einem ersten Schritt die wässerige Phase, die Ölphase und die Polymerpartikel unter Rühren zu vermischen und das erhaltene Gemisch in einem zweiten Schritt einer auf dem Prinzip der Kavitation basierenden Homogenisierung zu unterziehen.

20. Verfahren zur Herstellung nach Anspruch 19, dadurch gekennzeichnet, daß die Homogenisierung in dem zweiten Schritt bei einem Druck von 400 bis 700 bar durchgeführt wird.

21. Verwendung von Partikeln eines ionischen Polymers zur Stabilisierung einer fluiden Emulsion vom Öl-in-Wasser-Typ, die Ölkügelchen mit einer mittleren Größe von höchstens 500 Nanometer enthält.

22. Verwendung der Emulsion nach einem der Ansprüche 1 bis 11 zur photochemischen Stabilisierung eines chemischen Sonnenschutzfilters.

23. Verwendung nach Anspruch 22, dadurch gekennzeichnet, daß das Filter 2-Ethylhexyl-α-cyano-β,β-diphenylacrylat ist.
